# EUROPÄISCHE PATENTSCHRIFT

(11) **EP 1 721 560 B1**
(45) Veröffentlichungstag und Bekanntmachung des Hinweises auf die Patenterteilung: **11.06.2008**
(21) Anmeldenummer: 06110969.0
(22) Anmeldetag: 10.03.2006
(51) Int. Cl.: A61B 1/00

(54) **Diskontinuierlicher Linearantrieb**
Discontinuous linear drive
Entraînement linéaire discontinu

(30) Priorität: 12.05.2005 DE 102005022132
(43) Veröffentlichungstag der Anmeldung: 15.11.2006
(73) Patentinhaber: INVENDO MEDICAL GMBH, 69469 Weinheim (DE)
(72) Erfinder: Bob, Konstantin, 69469, Weinheim (DE); Pauker, Fritz, 86438, Kissing (DE)
(74) Vertreter: TBK-Patent

(56) Entgegenhaltungen:
- US-A- 6 048 307

## Beschreibung

Die Erfindung betrifft einen diskontinuierlichen Linearantrieb mit einem Führungsbauteil und einer Eingriffseinrichtung, die ein durch die Führungseinrichtung geführtes Element vorantreibt.

Aus dem Stand der Technik, beispielsweise gemäß der DE-OS 39 25 484 der Anmelderin selbst ist ein diskontinuierlicher Antrieb dieser Gattung bekannt. Bei diesem Antrieb sind zwei axial beabstandete Stützelemente in Form von radial aufweitbaren Stützringen vorgesehen, welche über ein hülsenförmiges Distanzstück miteinander gekoppelt sind. Das Distanzstück wird vornehmlich von einem Piezoelement oder einem hydraulisch betätigbaren Faltenbalg gebildet, der demzufolge in seiner Axiallänge veränderbar ist.

Für eine Fortbewegung ist es vorgesehen, die beiden Stützringe abwechselnd radial aufzuweiten und wieder zusammenzuziehen und dabei den gerade sich nicht abstützenden Stützring durch Ausdehen bzw. Zusammenziehen des Distanzstücks vorwärts zu drücken beziehungsweise nachzuziehen. Ein derartiger Linearantrieb zieht dabei eine Last oder ein zu beförderndes Bauteil beispielsweise in einen röhrenförmigen Hohlraum, wobei sich die axialen Stützringe an der Innenseite der Hohlraumwand abstützen.

Obgleich hierdurch ein gesteuerte Fortbewegung möglich ist, benötigt der bekannte diskontinuierliche Linearantrieb eine externe Abstützung, beispielsweise die vorstehend genannte Hohlraumwand, sodass dessen Einsatz nicht immer möglich ist bzw. nur nach kostspieligen Umbaumaßnahmen und Adaptionen an die räumlichen Gegebenheiten erfolgen kann.

Daher liegt der Erfindung die Aufgabe zugrunde, einen verbesserten und kostengünstigen Linearantrieb dieser Gattung bereitzustellen.

Diese Aufgabe wird durch einen diskontinuierlichen Linearantrieb mit den Merkmalen gemäß dem Patentanspruch 1 gelöst. Der Kern der Erfindung besteht demzufolge darin, dass der diskontinuierlicher Linearantrieb unter anderem ein Führungsbauteil und eine Eingriffseinrichtung hat, die mit einem durch das Führungsbauteil geführten anzutreibenden Element an zumindest zwei Stellen jeweils mittels eines linear bewegbaren Eingriffsbauteils (3a, 3k) in Eingriff bringbar ist. Hierbei wird das geführte Element durch phasenverschobenes Bewirken und Lösen des Eingriffs der bewegbaren Eingriffsbauteile (3a, 3k) angetrieben.

Das Führungsbauteil ist demnach bedingt durch das vorstehend beschriebene Antriebskonzept ortsfest und dient somit als interne Abstützung für den Vorwärtsantrieb. Der so ausgestaltete Linearantrieb arbeitet weitgehend unabhängig von örtlichen Gegebenheiten und muss folglich nicht an unterschiedliche Randbedingungen wie beispielsweise Innenabmessung eines zu explorierenden Hohlraums angepasst werden. Eine solche Standardausführung eines diskontinuierlichen Linearantriebs ist daher wesentliche kostengünstiger und funktioneller als die aus dem Stand der Technik bekannten Antriebe und eignet sich daher prinzipiell als Ein-Weg-Artikel.

Weitere vorteilhafte Ausgestaltungen der Erfindung sind Gegenstand der Unteransprüche.

Die Erfindung wird nunmehr nachfolgend anhand eines bevorzugten Ausführungsbeispiels unter Bezugnahme auf die begleitenden Zeichnungen näher erläutert.
- Fig. 1: zeigt den diskontinuierlichen Linearantrieb der vorliegenden Erfindung in einer teilweisen Explosionsansicht,
- Fig. 2: zeigt den diskontinuierlichen Linearantrieb von Fig. 1 aus einer anderen Perspektive und

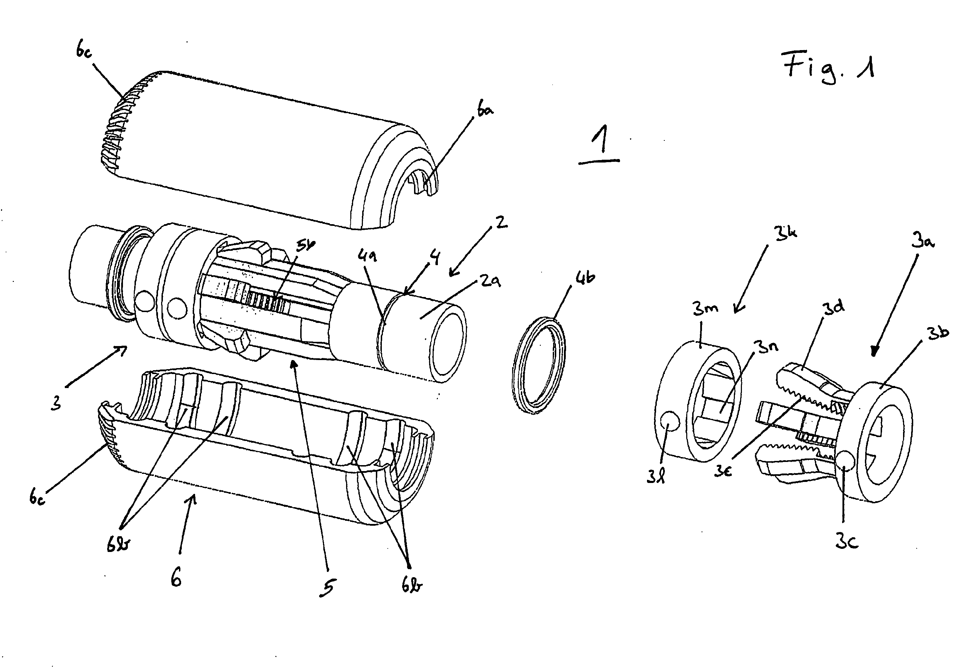

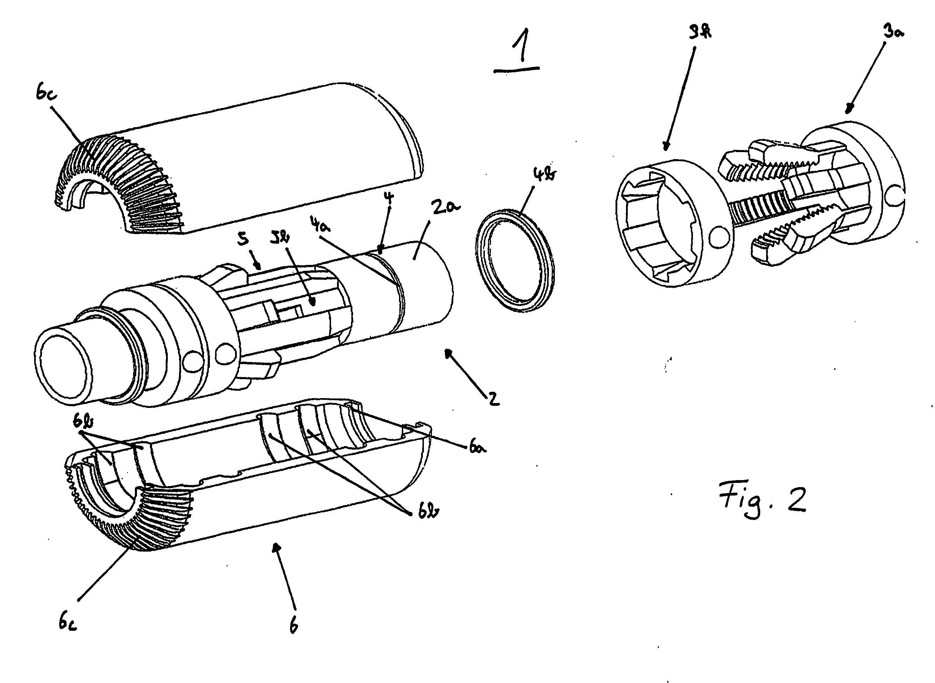

Der diskontinuierliche Linearantrieb 1 der vorliegenden Erfindung ist in den Figuren 1 und 2 in einer Explosionsansicht und von unterschiedlichen Perspektiven aus betrachtet dargestellt. Hergestellt werden sämtliche Bauteile des vorliegenden Linearantriebs vorzugsweise aus Kunststoffspritzguss, jedoch können ebenso weitere Materialien verwendet werden, die in ihrer Herstellung günstig sind und bei welchen sich lediglich ein einmaliger Einsatz aufgrund der günstigen Herstellungskosten rentiert.

Wie aus den Figuren entnommen werden kann, besteht der diskontinuierliche Linearantrieb 1 prinzipiell aus einem Führungsbauteil 2 und einer Eingriffseinrichtung 3.

Das Führungsbauteil 2 ist vorzugsweise als ein Rohrbauteil 2a bzw. als ein röhren- bzw. hülsenförmiges Bauteil ausgebildet. In dem Rohrbauteil 2a (bzw. in dessen Hohlraum) befindet sich ein anzutreibende Element (nicht gezeigt) beispielsweise der Schaft eines Endoskops oder ein diesen ummantelnder Stülpschlauch, welches durch den diskontinuierlichen Linearantrieb 1 angetrieben werden soll. Das Rohrbauteil 2a besteht vorzugsweise aus drei axial nacheinander aufgereihten Abschnitten, nämlich aus zwei axial beabstandeten Endabschnitte und einem dazwischenliegenden Mittelabschnitt. Die Außendurchmesser der beiden Endabschnitte sind im Wesentlichen die Gleichen, wohingegen der Mittelabschnitt einen geringfügig größeren Außendurchmesser aufweist. Der Übergang zwischen den beiden Endabschnitten zu dem Mittelabschnitt erfolgt dabei nicht abrupt sondern allmählich unter Ausbildung jeweils einer Konusform. Die Innendurchmesser aller drei Abschnitte sind im wesentlichen gleich.

Allerdings sind auch andere Übergangsformen als der vorstehend beschriebene lineare bzw. konusförmige Übergang denkbar. So kann ebenso ein konvex oder konkav verlaufender Übergang zwischen dem entsprechenden Endabschnitt und dem Mittelabschnitt in Betracht gezogen werden. Auch ist es möglich, dass alle drei genannten Abschnitte des Rohrbauteils 2a im wesentlichen gleiche Außendurchmesser aufweisen. Der Innendurchmesser des Rohrbauteils 2a hingegen bleibt in der bevorzugten Ausführungsform fortwährend über all die vorstehend genannten Abschnitte der Gleiche und ist demnach über das gesamte Rohrbauteil 2a konstant.

Die Endabschnitte des Rohrbauteils 2a sind jeweils mit einer Lagerungseinrichtung 4 ausgestattet, welche vorzugsweise eine in Umfangsrichtung des Rohrbauteils 2a bewegbare und eine in dessen Axialrichtung feststehende Lagerung darstellt.

Konkreter ausgedrückt ist die Lagerungseinrichtung 4 in der Form einer umlaufend an dem jeweiligen Endabschnitt des Rohrbauteils 2a befindlichen Nut 4a ausgebildet, wie in Figur 1 oder 2 ersichtlich ist. Weiter weist die Lagerungseinrichtung 4 einen in die Nut 4a eingesetzten Gleitring 4b auf, dessen Abmessungen derart gestaltet sind, dass ein Teil von diesem (ein äußerer Umfangsabschnitt des Gleitrings 4b) einen an dem Endabschnitt des Rohrbauteils 2a umlaufenden Vorsprung bzw. einen Wellenabsatz ausbildet. Auf die Funktionsweise der Lagerungseinrichtung 4 bzw. der Nut 4a und dem darin eingesetzten Gleitring 4b wird später im Detail eingegangen, wenn die hierfür relevanten, weiteren Einrichtungen bzw. Bauteile beschrieben sind.

Der Mittelabschnitt des Rohrbauteils 2a ist vorzugsweise so beschaffen, dass die vorstehend genannte Eingriffseinrichtung 3 daran in Axialrichtung des Rohrbauteils 2a beweglich anbringbar ist, worauf später im Detail eingegangen wird. Konkreter ausgedrückt ist der Mittelabschnitt des Rohrbauteils 2a mit einer Führungseinrichtung 5 zum Führen der Eingriffseinrichtung 3 vorgesehen. Diese ist im vorliegenden Ausführungsbeispiel in der Form von Aussparungen oder Schlitzen 5b ausbildet, die in Längsrichtung des Rohrbauteils 2a bzw. des Mittelabschnitts eine vorbestimmte Strecke, vorzugsweise über die gesamte Länge des Mittelabschnitts verlaufen. Vorzugsweise sind mehrere über den Umfang des Rohrbauteils 2a gleichmäßig beabstandete Aussparungen 5b vorgesehen. Da die Tiefe der Aussparungen 5b der Dicke (Rohrdicke) des Rohrbauteils 2a entspricht, stellen die Aussparungen 5b Durchgangsschlitze an dem Mittelabschnitt des Rohrbauteils 2a dar.

Die Gestalt der Durchgangsschlitze 5b ist beliebig. Vorzugsweise wird eine rechtecksförmige Gestalt ausgewählt. Ebenso ist aber auch ein derart gestalteter Schlitz denkbar, welcher an dessen beiden axialen Enden durch einen halbkreisförmigen Rand abgeschlossen wird.

In die vorstehend beschriebene Führungseinrichtung 5 bzw. in die oben erwähnten Durchgangsschlitze 5b ist die bereits erwähnte Eingriffseinrichtung 3 eingesetzt, die nachstehend im Detail beschrieben wird.

Die durch die Führungseinrichtung 5 bzw. durch die Durchgangsschlitze 5b geführte Eingriffseinrichtung 3 besteht im Wesentlichen aus zumindest zwei Eingriffsbauteilen, wovon jedes ein Eingreifelement 3a und ein Klemmelement 3k aufweist.

Bevorzugt ist das Eingreifelement 3a zumindest teilweise in einer ringförmigen Gestalt vorgesehen, wodurch ein Ringabschnitt 3b des Eingreifelements 3a ausgebildet wird. Der Innendurchmesser des Ringabschnitts 3b des Eingreifelements 3a entspricht im Wesentlichen (geringes Übermaß) dem Außendurchmesser eines entsprechenden Endabschnitts des Rohrbauteils 2a, wodurch das Eingreifelement 3a auf das Rohrbauteil 2a gleitend aufsteckbar ist. Ebenso kann dessen Innendurchmesser auch demjenigen des Mittelabschnitts entsprechen. Hierauf wird später im Detail eingegangen.

Der Ringabschnitt 3b des Eingreifelements 3a weist einen Mitnehmer, vorzugsweise einen Vorsprung bzw. Nocken 3c, an dessen Außenumfang auf, welcher in der Gestalt einer Halbkugel ausgebildet sein kann. Ebenso sind aber auch andere Ausgestaltungen denkbar, wie beispielsweise einen radial vorstehenden Stift oder ein negativer Mitnehmer wie beispielsweise eine in Umfangsrichtung des Ringabschnitts 3b verlaufende Außennut oder Rille, solange der hierfür bestimmte Verwendungszweck erreicht werden kann, wie später im Detail beschrieben wird.

Weiter weist das Eingreifelement 3a neben dem Ringabschnitt 3b einen Klemmabschnitt 3d auf. Der Klemmabschnitt 3d besteht aus zumindest einem in Achsrichtung des Ringabschnitts 3b verlaufenden, ggf. keilförmigen Klemmfinger oder Klaue, dessen Wurzel an der Stirnseite des Ringabschnitts 3b entspringt. Vorzugsweise bildet der Klemmabschnitt 3d eine Vielzahl von Klemmfingern aus, die in Umfangsrichtung des Ringabschnitts 3b des Eingreifelements 3a gleichmäßig beabstandet sind. Insbesondere entspricht die Anzahl und Beabstandung der einzelnen Klemmfinger sowie deren Breite der Anzahl und der Beabstandung der Schlitze sowie deren Breite (Aussparungen 5b) der Führungseinrichtung 5. Weiter sind die Klemmfinger derart dimensioniert, dass diese problemlos in die Schlitze eingesetzt werden können, worauf später im Detail eingegangen wird.

Die den Klemmabschnitt 3d ausbildenden Klemmfinger sind so ausgebildet, dass diese vorzugsweise elastisch flexibel in radialer Richtung bezüglich des Ringsabschnitts 3b sind. Hierfür weist jeder Klemmfinger im Bereich seiner Wurzel eine Sollbiegestelle auf, welche durch eine Wandausdünnung in Radialrichtung erzeugt wird D.h., dass die Klemmfinger jederzeit in gewissen Grenzen elastisch verbogen werden können und nach der Biegung wieder zu ihr ursprünglichen Stellung selbständig zurückkehren. Der Querschnitt jedes Klemmfingers nimmt vorzugsweise nahezu linear von seiner Wurzel in Richtung zu seinem freien Ende zu. Dadurch ist jeder Klemmfinger in Axialrichtung betrachtet in der Form eines Keils ausgebildet. Ebenso verlaufen die Klemmfinger in Achsrichtung des Ringabschnitts 3b derart, dass diese in Richtung hin zu deren freien Enden zunehmend radial nach außen stehen. Als eine alternative Ausführungsform zu den keilförmigen Klemmfingern, sind derart dimensionierte Klemmfinger denkbar, die keine stetigen Querschnittsverlauf haben (wie der keilförmige Klemmfinger), sondern aus einem Abschnitt mit geringen Querschnitt und einem daran angrenzenden Abschnitt mit großem Querschnitt bestehen, so das eine quasi sprungartige Querschnittserhöhung stattfindet. Bevorzugt ist jedoch, dass der Klemmabschnitt 3d aus einer Vielzahl von keilförmigen Klemmfingern ausgebildet ist, um einen Keileffekt ausnutzen zu können, worauf später genauer eingegangen wird.

Jeder keilförmige Klemmfinger weist an seiner radial inneren Seite sowie im Bereich seines freien Endes eine Eingriffsfläche 3e auf, wie dies in Figur 1 gezeigt ist. Die Eingriffsfläche 3e ist im Allgemeinen derart beschaffen, dass diese eine hohe Rauhigkeit aufweist, d.h. bei einem Eingriffszustand (bei Kontaktaufnahme) mit dem anzutreibenden Element einen hohen Reibungskoeffizienten erzeugt. Vorzugsweise wird eine Verzahnungsstruktur für die Eingriffsfläche 3e gewählt, die ähnlich der Struktur beispielsweise einer Zahnstange ist. Die radial äußere Fläche jedes keilförmigen Klemmfingers ist im Gegensatz zu der Eingriffsfläche sehr glatt gestaltet, um jegliche Reibung zwischen dieser und einer anderen Fläche bzw. einem anderen Bauteil zu minimieren d.h. um eine Gleitfläche zu bilden.

Schließlich weist das Eingriffsbauteil 3 neben dem Eingreifelement 3a ein Klemmelement 3k auf.
Das Klemmelement 3k hat vorliegend eine ringförmige Gestalt, ist an dem Außenumfang mit einem Mitnehmer, vorzugsweise einem Vorsprung oder Nocke 31 (in gleicher Weise modifizierbar wie der Vorsprung 3c des Ringabschnitts 3b des Eingreifelements) vorgesehen, der gleich oder ähnlich wie der vorstehend erwähnte Vorsprung 3c des Ringabschnitts 3b des Eingreifelements 3a ausgebildet sein kann, und hat an dessen Innenumfang eine Vielzahl von in Axialrichtung des ringförmigen Klemmelements 3k verlaufende Nuten 3n (bzw. Aussparungen) ausgebildet, die entsprechend den keilförmigen Klemmfingern des Eingreifelements 3a gleichmäßig über den Umfang beabstandet sind. Die Nuten 3d verlaufen am Innenumfang des Klemmelements 3k über dessen gesamte Länge und können vorzugsweise bezüglich der Axialrichtung des ringförmigen Klemmelements 3k an dessen Nutgrund konisch abgeschrägt sein, um den Keileffekt weiter auszunutzen, wie später im Detail beschrieben wird. Weiter sind die Abmessungen des Klemmelements 3k vorzugsweise denen des Ringabschnitts 3b des Eingreifelements 3a identisch, d.h. gleichen Innen- und Außendurchmesser (mit geringem Übermaß) etc.

Das Eingreifelement 3a und das Klemmelement 3k eines Eingriffsbauteils der Eingriffseinrichtung 3 sind entsprechend dem vorstehend beschriebenen Aufbau so zueinander angeordnet, dass der Ringabschnitt 3b des Eingreifelements 3a und das ringförmige Klemmelement 3k axial aneinander angrenzend und konzentrisch angeordnet sind, wobei der in der Form von keilförmigen Klemmfingern ausgebildete Klemmabschnitt 3d in die entsprechenden Nuten 3n des Klemmelements 3k eingesetzt ist. Somit kann aufgrund der vorzugsweise elastischen Biegbarkeit der keilförmigen Klemmfinger des Eingreifelements 3a, die in die jeweiligen Nuten 3n des Klemmelements 3k eingesetzt sind, das ringförmige Klemmelement 3k relativ zu dem Eingreifelement 3a in dessen Axialrichtung verschoben werden, wobei dieses durch die keilförmigen Klemmfinger und die Nuten 3n geführt wird.

Das Klemmelement 3k und das Eingreifelement 3a, die derart zueinander angeordnet sind, stellen ein einzelnes Eingriffsbauteil dar, wovon die Eingriffseinrichtung 3 zumindest zwei aufweist, wie dies vorstehend erwähnt wurde. Die zumindest zwei Eingriffsbauteile werden durch die Führungseinrichtung 5 bzw. durch die Aussparungen 5b (Längsschlitze) des Rohrbauteils 2a in dessen Axialrichtung bewegbar geführt. Das heißt im Detail, dass das Eingriffsbauteil bei der Führungseinrichtung 5 an dem Mittelabschnitt des Rohrbauteils 2a angeordnet ist. Ebenso ist aber auch eine Anordnung denkbar, bei welcher das entsprechende Eingriffsbauteil, je nach dessen Dimensionierung, etwa bei dem Übergang zwischen dem entsprechenden Endabschnitt und dem Mittelabschnitt angeordnet ist.

Die keilförmigen Klemmfinger des Eingreifelements 2a befinden sich in den entsprechend ausgebildeten Aussparungen 5b (Längsschlitze) der Führungseinrichtung 5, wodurch die keilförmigen Klemmfinger des Eingreifelements 3a entsprechend dem Verlauf der Längsschlitze in der Axialrichtung des Rohrelements geführt werden. Somit ist jedes Eingriffsbauteil der Eingriffseinrichtung 3 in Axialrichtung des Rohrbauteils 2a bewegbar angeordnet. Weiterhin sind aber auch das Eingreifelement 3a und das Klemmelement 3k, wie aus dem vorstehend erwähnten Aufbau hervorgeht, relativ zueinander beweglich angeordnet, da sich die keilförmigen Klemmfinger des Eingreifelements 3a im Gleiteingriff mit den entsprechenden Nuten 3n des Klemmelements 3k befinden bzw. in die entsprechenden führenden Nuten 3n des Klemmelements 3k gleitfähig eingesetzt sind.

Zum Antreiben und Steuern der Bewegung der beiden Eingriffsbauteile, die im Axialabstand zueinander beweglich an der Führungseinrichtung 5 angebracht sind, weist der diskontinuierliche Linearantrieb 1 gemäß der Erfindung eine Antriebs-/Bewegungssteuerungseinrichtung 6 auf. Diese Antriebseinrichtung 6 ist in der Form eines Hohlzylinders bzw. einer Manschette mit einer zumindest innenseitigen Kulisse ausgebildet. Die manschettenförmige Antriebseinrichtung 6 ist konzentrisch zum Rohrbauteil 2a angeordnet und umgibt dieses. Folglich befindet sich das Rohrbauteil 2a in dem Hohlraum der manschettenförmigen Antriebseinrichtung 6. Weiter weist die manschettenförmige Antriebseinrichtung 6 an dessen beiden axialen Enden einen Lagerabschnitt 6a auf, der mit der entsprechenden Lagerungseinrichtung 4 des Führungsbauteils 2 in Verbindung steht. Dadurch wird die manschettenförmige Antriebseinrichtung 6 drehbar um dessen Achse am Rohrbauteil 2a gelagert, ist aber in dessen Axialrichtung zum Rohrbauteil unbeweglich.

Im Falle der vorstehend beschriebenen Ausführungsform, wonach die Lagerungseinrichtung 4 in der Form der vorstehend genannten Nut 4a an dem Endabschnitt des Rohrbauteils 2a ausgebildet ist, in die der vorstehend erwähnte Gleitring 4b eingesetzt ist, ist der jeweils an einem axialen Ende der manschettenförmigen Antriebseinrichtung 6 befindliche Lagerabschnitt 6a ebenfalls in der Form einer um den Innenumfang der manschettenförmigen Antriebseinrichtung 6 verlaufenden Nut ausgebildet, in die der überstehende Abschnitt (äußere Umfangsabschnitt) des in die Nut 4a eingesetzten Gleitrings 4b eingesetzt ist. Somit ist eine bevorzugte Art der Lagerung zwischen der manschettenförmigen Antriebseinrichtung 6 und der Lagerungseinrichtung 4 beschrieben, es sind jedoch andere Lagerungsarten ebenso möglich, solange die manschettenförmige Antriebseinrichtung 6 drehbar an dem Rohrbauteil 2a angebracht ist.

Weiter weist die manschettenförmige Antriebseinrichtung 6 eine am Innenumfang befindliche Kulisse oder nachfolgend auch Bewegungssteuerungseinrichtung 6b genannt, auf, welche in der bevorzugten Ausführungsform als eine Vielzahl (vorliegend vier entsprechend der Anzahl von Eingriffs- und Klemmelementen) am Innenumfang umlaufende Nut- oder Nockenkurven vorgesehen ist. Die einzelnen Nockenkurven sind nacheinander in einer Axialrichtung der manschettenförmigen Antriebseinrichtung 6 ausgebildet, wobei jede Einzelne einen geschlossen, quasi ringförmigen Verlauf hat. Konkreter ausgedrückt ist jede der Nockenkurven so ausgebildet, dass sie mit einem entsprechenden Mitnehmer einer der Eingriffs- oder Klemmelemente in Gleiteingriff steht, um bei einer Rotation der Manschette eine Axialbewegung des jeweiligen in Gleiteingriff stehenden Eingriffs- oder Klemmelements zu bewirken. In sofern wird der Bewegungshub sowie die Bewegungsphase jedes der Eingriffs- oder Klemmelemente über die einzelnen Nuten in der Manschette bestimmt. Auf die genaue konstruktive Beschreibung des Verlaufs jeder Nockenkurve wird an dieser Stelle verzichtet und auf den folgenden Funktionsbeschreibungsteil der vorliegenden Erfindung verwiesen. Es sei nur erwähnt, dass im vorliegenden bevorzugten Ausführungsbeispiel die Manschette aus zwei Halbschalen gebildet ist, die bei der Montage am Rohrbauteil zusammengefügt werden, wobei jede Halbschale innenseitig die vorstehend genannte Kulissenführung aufweist. Auf diese Weise können unterschiedliche Bewegungshübe und Bewegungsphasen durch Austausch bzw. Kombination einzelner Manschettenhalbschalen bewirkt werden.

Bei der vorliegendenden Erfindung stehen jeweils zwei in Axialrichtung der manschettenförmigen Antriebseinrichtung 6 nebeneinander angeordnete Nockenkurven jeweils mit den vorzugsweise halbkugelförmigen Mitnehmern des Eingreifelements 3a und des zugehörigen Klemmelements 3k, welche ein Eingriffsbauteil bilden, in Gleiteingriff. An dieser Stelle sei darauf hingewiesen, dass wie bei den Modifizierungen der Mitnehmer 3c und 3l des Eingreifelements 3a und des Klemmelements 3k auch hier entsprechende Ausgestaltungen der Nockenkurven vorgenommen werden können, solange der entsprechende Eingriff bzw. Gleiteingriff hergestellt werden kann. So kann die Vielzahl von Nockenkurven beispielsweise auf der Außenseite der Eingreifelemente und Klemmelemente ausgebildet sein wohingegen an der Innenseite Manschette ein Eingriffsvorsprung radial nach innen vorsteht.

Die Anzahl der Nockenkurven ist hierbei, wie bereits schon angedeutet wurde, abhängig von der Anzahl der Eingriffsbauteile. Bei der vorliegenden Erfindung bedeutet dies, das bei der Verwendung von zumindest zwei Eingriffsbauteilen zumindest vier Nockenkurven an dem Innenumfang der manschettenförmigen Antriebseinrichtung 6 umlaufend vorgesehen sind, die mit den entsprechenden halbkugelförmigen Mitnehmern der Eingriffsbauteile in Eingriff stehen.

Um die vorstehend geschilderte manschettenförmige Antriebseinrichtung in Rotation zu versetzen, weist diese in der bevorzugten Ausführungsform eine an deren Umfang ausgebildete Verzahnung (Zahnkranz 6c) auf, die mit einem Getriebe oder dergleichen im Eingriff steht, welches beispielsweise von einem Motor (nicht gezeigt) angetrieben wird. Darüber hinaus ist nicht notwendiger Weise eine Verzahnung 6c vorzusehen. Jegliche andere Einrichtung, die die manschettenförmige Antriebseinrichtung 6 in Drehung versetzen kann, ist geeignet. So kann beispielsweise ebenso ein Riemenantrieb oder dergleichen hierfür verwendet werden.

Im Folgenden wird der Betrieb des diskontinuierlichen Linearantriebs 1 ausführlich beschrieben.

Um den diskontinuierlichen Linearantrieb zu betreiben, wird die Antriebseinrichtung 6 in Bewegung versetzt. Im Falle der manschettenförmigen Antriebseinrichtung 6, welche drehbar um dessen Längsachse mittels der entsprechenden Lagerabschnitte 6a und der Lagerungseinrichtungen 4 des Rohrbauteils 2a gelagert ist, wird diese durch den Motor, das Getriebe oder dergleichen in eine Drehbewegung versetzt, während sich das Rohrbauteil 2a in einer drehfesten Lage befindet. Durch die Rotation der manschettenförmigen Antriebseinrichtung 6 um das Rohrbauteil wird mittels der Bewegungssteuerungseinrichtung 6b der Antriebseinrichtung 6 die Eingriffseinrichtung angetrieben. Im Falle der als Nockenkurven ausgebildeten Bewegungssteuerungseinrichtung 6b werden die mit diesen in Eingriff bzw. Gleiteingriff stehenden, vorzugsweise halbkugelförmigen Mitnehmer 3c und 3l der jeweiligen Eingriffsbauteile geführt, d.h. die Rotationsbewegung der manschettenförmigen Antriebseinrichtung 6 wird in eine translatorische Bewegung des entsprechenden Eingriffsbauteils bzw. der Elemente des jeweiligen Eingriffsbauteils umgewandelt (vorzugsweise zwei Eingriffsbauteile), deren Richtung der Axialrichtung des Rohrbauteils entspricht. Da zumindest zwei Eingriffsbauteile der Eingriffseinrichtung bei dem diskontinuierlichen Linearantrieb 1 verwendet werden, sind je Eingriffsbauteil zwei Nockenkurven vorgesehen, wovon jeweils Eine mit dem halbkugelförmigen Mitnehmer 3c des Eingreifelements 3a und die Andere mit dem halbkugelförmigen Mitnehmer 31 des Klemmelements 3k in Gleiteingriff steht.

Um den Verlauf der entsprechenden Nockenkurven möglichst einfach zu gestalten, ist es von Vorteil, die Elemente eines Eingriffsbauteils derart anzuordnen, dass deren halbkugelförmigen Mitnehmer 3c, 31 jeweils am Außenumfang des Ringabschnitts 3b und am Außenumfang des Klemmelements 3k einander gegenüberstehend angeordnet sind, d.h. die selbe Radialposition und Winkelposition einnehmen.

Durch die als Längsschlitze ausgebildete Führungseinrichtung 5, welche vorzugsweise gleichmäßig über den Umfang des Rohrbauteils beabstandet sind, sind beide Eingriffsbauteile drehfest gehalten, da die keilförmigen Klemmfinger 3d des Eingreifelements 3a in die Längsschlitze eingesetzt sind. Ebenso ist auch das Klemmelement 3k drehfest gehalten, da die an dessen Innenumfang befindlichen Nuten 3n mit den keilförmigen Klemmfingern des Eingreifelements 3a in Eingriff stehen. Folglich ist lediglich eine Bewegung des Eingriffsbauteils in Axialrichtung des Rohrbauteils 2a möglich, wobei auch das Eingreifelement 3a und das Klemmelement 3k relativ zueinander in Axialrichtung des Rohrbauteils 2a aufgrund der vorstehend erwähnten Nut-Finger-Verbindung bewegbar sind.

Somit kann der Verlauf der Nockenkurven sowohl die Axialbewegung eines Eingriffsbauteils als Ganzes als auch die Bewegung der beiden Elemente des Eingriffsbauteils, des Eingreifelements 3a und des Klemmelements 3k, relativ zueinander steuern.

Demnach werden beide Elemente eines Eingriffsbauteils durch den Verlauf der entsprechenden Nockenkurven zum Einen voneinander wegbewegt als auch wieder zueinander hinbewegt. Werden beide Elemente voneinander wegbewegt, führt dies dazu, das die ursprünglich radial nach außen gebogenen bzw. stehenden keilförmigen Klemmfinger mittels der Nuten 2n des Klemmelements 3k umso weiter radial nach innen gedrückt werden, je weiter diese voneinander wegbewegt werden (Keileffekt). Demnach wird durch Relativbewegung der beiden Elemente eines Eingriffsbauteils der Keileffekt ausgenutzt, dessen Ausmaß von der Gestaltung der keilförmigen Klemmfinger und den vorstehend genannten, gegebenenfalls abgeschrägten Flächen der Nuten 3n abhängt. Kehrt sich der Verlauf der Nockenkurven um, so dass beide Element zueinander bewegt werden, kehren die keilförmigen Klemmfinger des Eingreifelements 3a aufgrund deren Elastizität zu deren ursprünglichen Position zurück, also wieder weiter radial nach außen stehend.

Bei voneinander beabstandeten Elementen eines Eingriffsbauteils werden die keilförmigen Klemmfinger des Eingreifelements 3a durch die Längsschlitze gedrückt und ein Eingriffszustand mit dem anzutreibenden Bauteil wie Endoskopschaft ist hergestellt, indem die Eingriffsflächen 3e das in dem Rohrbauteil 2a befindliche, anzutreibende Element greifen. Der Eingriffszustand wird wieder aufgehoben bzw. gelöst, wenn beide Elemente zueinander bewegt werden, so dass diese aneinander angrenzen.

Somit kann durch Steuerung des Abstands der beiden Elemente eines Eingriffsbauteils der Eingriffszustand hergestellt und aufgehoben werden. Wird der Abstand beider Elemente eines Eingriffsbauteils nicht verändert, sondern beide Elemente gleichförmig bzw. parallel zueinander bewegt, so wird der entsprechende Zustand aufrechterhalten und entweder das anzutreibende Element bewegt, falls der Eingriffszustand besteht, oder das Eingriffselement bewegt, ohne das anzutreibende Element zu bewegen, nämlich im Falle des gelösten Zustands.

Somit können bei geeignetem Verlauf der Nockenkurven und entsprechender Phasenverschiebung der hierdurch bestimmten Bewegungshübe, die Eingriffsbauteile derart gesteuert werden, dass diese abwechselnd bzw. phasenverschoben den Eingriffzustand herstellen und sich unter Aufrechterhaltung des Eingriffszustands entlang der Axialrichtung des Rohrbauteils 2a bewegen, wodurch das anzutreibende Element voranbewegt wird. Das heißt im Detail, dass während das eine Eingriffsbauteil so gesteuert wird, dass dieses das anzutreibende Element greift und sich in eine Längsrichtung des Rohrbauteils 2a bewegt, sich das andere Eingriffsbauteil in einem gelösten Zustand befindet und sich dabei entgegengesetzt bewegt. Sobald das eine Eingriffsbauteil entsprechend der Nockenkurven das anzutreibende Element um einen vorbestimmten Hub bewegt hat (abhängig von dem Verlauf der Nockenkurven) und sich im Übergang zum gelösten Zustand befindet, befindet sich das andere Eingriffsbauteil durch den Verlauf der Nockenkurven im Übergang zum Eingriffszustand, wodurch das anzutreibende Bauteil wechselweise von den beiden Eingriffsbauteilen um einen vorbestimmten Hub voranbewegt bzw. angetrieben wird. Daher kann der Verlauf der Nockenkurven derart beschrieben werden, dass diese ein Eingriffsbauteil im Eingriffszustand halten und aufgrund der Rotation der manschettenförmigen Antriebseinrichtung 6 um einen vorbestimmten Hub in eine Längsrichtung des Rohrbauteils bewegen. Sobald das eine Eingriffsbauteil seinen Eingriffszustand löst und das Andere seine Eingriffzustand herstellt, verlaufen die Nockenkurven derart, das diese das eine im gelösten Eingriffszustand befindliche Eingriffsbauteil an dessen ursprüngliche Position zurückführen. Folglich Verlaufen die Nockenkurven derart, dass diese ihren Abstand voneinander nicht ändern, wenn ein Zustand aufrechterhalten werden soll, und ihren Abstand von einander ändern (in die Richtung des entsprechenden Zustands), wenn der derzeitige Zustand geändert werden soll.

Es sei angemerkt, dass die Antriebsrichtung des anzutreibenden Bauteils von der Drehrichtung der manschettenförmigen Antriebseinrichtung 6 abhängt, d.h. dass bei einer Umkehr der Drehrichtung der manschettenförmigen Antriebseinrichtung 6 sich ebenso die Antriebsrichtung des anzutreibenden Bauteils umkehrt.

Wie bereits vorstehend erwähnt ist, ist die Anzahl der Eingriffsbauteile nicht auf zwei beschränkt. Es können ebenso mehr als zwei Eingriffsbauteile verwendet werden, solange das vorstehend beschriebene Schema aufgrund des Verlaufs der Nockenkurven eingehalten wird. So können beispielsweise im Falle von vier Eingriffsbauteilen zwei Eingriffsbauteile gleichzeitig mit dem anzutreibenden Element im Eingriff stehen und dieses um einen vorbestimmten Hub voranbewegen, während die anderen beiden sich in einem gelösten Zustand befinden. Der weitere Ablauf erfolgt wie bereits beschrieben analog.

Auf diese Weise wird das anzutreibende Element nahezu stetig um vorbestimmte Hübe vorangetrieben, wobei der vorstehend beschriebene Antrieb vorzugsweise bei Stülpschlauchkonstruktionen für Endoskope eingesetzt wird. Das heißt, dass der Stülpschlauch samt Endoskopschaft eines Endoskops in das Rohrbauteil 2a eingeführt wird und somit ein Endoskopantrieb, der den Stülpschlauch kontinuierlich umgreift, gegeben ist.

Wie vorstehend beschrieben ist, wird das Steuern der Zustände des jeweiligen Eingriffsbauteils rein mechanisch durchgeführt, nämlich mittels der Steuerung des Abstands der beiden Elemente eines Eingriffsbauteils unter Ausnutzung des Keileffekts. Elektronische Steuerungseinrichtungen können somit entfallen.
Eine weitere alternative Ausführungsform ist es, die oben genannten Zustände fluidbetrieben zu steuern. So wird das entsprechende Eingreifelement mittels der zugehörigen Nockenkurve bewegt, und anstelle des mechanischen Klemmelements wird durch Steuerung eines Fluiddrucks auf die jeweiligen Vorsprünge zum Herstellen des Eingriffszustands ein entsprechender Druck aufgebracht. Das Prinzip der fluidbetriebenen Drucksteuerung ist analog zum oben beschrieben Prinzip. Daher wird auf eine ausführliche Beschreibung hiervon verzichtet.

Ein diskontinuierlicher Linearantrieb weist ein Führungsbauteil und eine Eingriffseinrichtung auf. Die Eingriffseinrichtung greift an einem durch das Führungsbauteil geführten und anzutreibenden Element an zumindest zwei Stellen jeweils mittels eines bewegbaren Eingriffsbauteils an. Das anzutreibende Element wird durch phasenverschobenes Bewirken und Lösen des Eingriffs der bewegten Eingriffsbauteile angetrieben.

## Patentansprüche

1. Diskontinuierlicher Linearantrieb mit einem Führungsbauteil (2) und einer Eingriffseinrichtung (3), die mit einem durch das Führungsbauteil (2) geführten anzutreibenden Element an zumindest zwei Stellen jeweils mittels eines linear bewegbaren Eingriffsbauteils (3a, 3k) in Eingriff bringbar ist und das geführte Element durch phasenverschobenes Bewirken und Lösen des Eingriffs der bewegbaren Eingriffsbauteile (3a, 3k) antreibt,
wobei die zumindest zwei
Eingriffsbauteile der Eingriffseinrichtung (3) von einer Antriebseinrichtung (6) bewegt werden und wobei die Antriebseinrichtung (b) in Form einer das Führungsbauteil (2) umgebenden, drehbar gelagerten Manschette mit einer innenseitigen Kulisse ausgebildet ist.

2. Diskontinuierlicher Linearantrieb gemäß Anspruch 1, **dadurch gekennzeichnet, dass** die zumindest zwei Eingriffsbauteile (3a, 3k) jeweils aus einem Eingreifelement (3a) und einem Klemmelement (3k) bestehen deren unterschiedliche Stellungen zueinander entweder einen Eingriff herstellen oder diesen lösen.

3. Diskontinuierlicher Linearantrieb gemäß Anspruch 1 **dadurch gekennzeichnet dass** die zumindest zwei Eingriffsbauteile jeweils aus einem Eingreifelement (3a) und einem Klemmelement (3k) bestehen, die einen Eingriffszustand bei einer auseinandergerückten Stellung herstellen und den Eingriffszustand bei zusammengerückter Stellung lösen.

4. Diskontinuierlicher Linearantrieb gemäß Anspruch 3, **dadurch gekennzeichnet, dass** die Bewegungssteuerungseinrichtung (6b) oder Kulisse der Antriebstriebseinrichtung (6) so ausgebildet ist, dass sie die Eingriffsbauteile als Ganzes bewegt und die Stellung der Elemente eines Eingriffsbauteils zueinander verändert.

5. Diskontinuierlicher Linearantrieb gemäß Anspruch 3, **dadurch gekennzeichnet, dass** die Kulisse als eine Vielzahl von Nockenkurven ausgebildet ist, die am Innenumfang umlaufend an der manschettenförmigen Antriebseinrichtung (6) ausgebildet ist und mit einem entsprechend geformten Gleitabschnitt, vorzugsweise einem Mitnehmer des Eingreifelements (3a) und des Klemmelements (3k) eines gleichen Eingriffsbauteils im Gleiteingriff steht.

6. Diskontinuierlicher Linearantrieb gemäß Anspruch 3, **dadurch gekennzeichnet, dass** das Eingreifelement (3a) einen Ringabschnitt (3b) und einen Klemmabschnitt (3d) aufweist, wobei der Klemmabschnitt (3d) als eine Vielzahl von keilförmigen und biegbaren Klemmfingern ausgebildet ist, die in Axialrichtung bezüglich des Ringabschnitts (3b) hervorstehen und die aufgrund deren keilförmigen Gestalt bezüglich des Ringabschnitts (3b) geringfügig radial nach außen gebogen sind.

7. Diskontinuierlicher Linearantrieb gemäß Anspruch 6, **dadurch gekennzeichnet, dass** das Klemmelement (3k) ringförmig ausgebildet ist, an dessen Innenumfang eine Vielzahl von Nuten (3n) vorgesehen ist, in die die Vielzahl der keilförmigen Mitnehmer des Eingreifelements (3a) eingesetzt ist.

8. Diskontinuierlicher Linearantrieb gemäß Anspruch 7, **dadurch gekennzeichnet, dass** das Rohrbauteil (2a) eine Führungseinrichtung (5) aufweist, welche in der Form von einer Vielzahl von Aussparungen (5b), vorzugsweise als Längsschlitze, ausgebildet ist, die an einem Abschnitt des Rohrbauteils (2a) über dessen Umfang verteilt gelegen sind, in die die Vielzahl der keilförmigen Klemmfingern des Eingreifelements (3a) eingesetzt ist.

9. Diskontinuierlicher Linearantrieb gemäß einem der vorhergehenden Ansprüche, **dadurch gekennzeichnet, dass** dieser vollständig Kunststoffspritzguss hergestellt ist.

10. Diskontinuierlicher Linearantrieb gemäß einem der vorhergehenden Ansprüche, **dadurch gekennzeichnet, dass** das Element, welches durch das als Rohrbauteil (2a) ausgebildete Führungsbauteil (2) geführt wird, eine Stülpschlauchkonstruktion mit Endoskopschaft ist, die durch den Linearantrieb angetrieben wird.

## Claims

1. A discontinuous linear drive comprising a guiding component (2) and an engaging means (3) which is adapted to be engaged with a member to be driven, guided by the guiding component (2), at at least two positions by means of a linearly movable engaging component (3a, 3k), respectively, and which drives said guided member by a phase-shifted effecting and releasing of the engagement of the movable engaging components (3a, 3k),
wherein the at least two engaging components of the engaging means (3) are moved by a drive means (6), and
wherein
the drive means (6) is provided in the form of a rotatably supported collar surrounding the guiding component (2), said collar being formed with an internal connecting member.

2. A discontinuous linear drive according to claim 1, **characterized in that** each of the at least two engaging components (3a, 3k) comprises an engaging member (3a) and a clamping member (3k) whose different positions with respect to each other either bring about or release an engagement.

3. A discontinuous linear drive according to claim 1, **characterized in that** each of the at least two engaging components comprises an engaging member (3a) and a clamping member (3k) which establish an engaged state in the case of a moved-apart position and release the engaged state in the case of a moved-together position.

4. A discontinuous linear drive according to claim 3, **characterized in that** the motion control means (6b) or connecting member of the drive means (6) is formed such that it moves the engaging components as a whole and varies the position of the members of an engaging component with respect to each other.

5. A discontinuous linear drive according to claim 3, **characterized in that** the connecting member is in the form of a plurality of cam curves peripherally formed at the inner circumference of the collar-shaped drive means (6) and being in sliding engagement with a correspondingly shaped sliding portion, preferably a driving dog of the engaging member (3a) and of the clamping member (3k) of an identical engaging component.

6. A discontinuous linear drive according to claim 3, **characterized in that** the engaging member (3a) includes an annular portion (3b) and a clamping portion (3d), the clamping portion (3d) being in the form of a plurality of wedge-shaped and bendable clamping fingers which project in an axial direction with respect to the annular portion (3b) and which are bent slightly radially outwardly with respect to the annular portion (3b) by virtue of their wedge-shaped design.

7. A discontinuous linear drive according to claim 6, **characterized in that** the clamping member (3k) has an annular shape at the inner circumference of which a plurality of grooves (3n) are provided in which the plurality of wedge-shaped driving dogs of the engaging member (3a) are inserted.

8. A discontinuous linear drive according to claim 7, **characterized in that** the tubular component (2a) has a guide means (5) in the form of a plurality of recesses (5b), preferably as longitudinal slits, which are arranged at a portion of the tubular component (2a) distributed along the circumference thereof, with the plurality of wedge-shaped clamping fingers of the engaging member (3a) being inserted in said recesses.

9. A discontinuous linear drive according to any of the preceding claims, **characterized in that** it is completely manufactured by plastic injection molding.

10. A discontinuous linear drive according to any of the preceding claims, **characterized in that** the member guided by the guiding component (2) being formed as a tubular component (2a) is an everting tube construction comprising an endoscope shaft, which is driven by the linear drive.

## Revendications

1. Dispositif d'entraînement linéaire discontinu, avec un composant de guidage (2) et un dispositif de mise en prise (3), susceptible d'être mis en prise avec un élément à entraîner, guidé au moyen du composant de guidage, en au moins deux emplacements, chaque fois au moyen d'un composant de mise en prise (3a, 3k) déplaçable de façon linéaire, et entraîne l'élément guidé, par provocation et cessation déphasées de la mise en prise des composants de mise en prise (3a, 3k) déplaçables,
où les au moins deux composants de mise en prise (3a, 3k) du dispositif de mise en prise (3) sont déplacés par un dispositif d'entraînement (6) et où le dispositif d'entraînement (6) est réalisé sous la forme d'une manchette, montée à rotation, entourant le composant de guidage (2), avec une coulisse située du côté intérieur.

2. Dispositif d'entraînement linéaire discontinu selon la revendication 1, **caractérisé en ce que** les au moins deux composants de mise en prise (3a, 3k) sont chacun composés d'un élément de mise en prise (3a) et d'un élément de serrage (3k), dont les différentes positions mutuelles, soit établissent une mise en prise, soit la font cesser.

3. Dispositif d'entraînement linéaire discontinu selon la revendication 1, **caractérisé en ce que** les au moins deux composants de mise en prise sont chacun composés d'un élément de mise en prise (3a) et d'un élément de serrage (3k), qui établissent un état de mise en prise dans une position mutuellement écartée, et font cesser l'état de mise en prise dans une position mutuellement regroupée.

4. Dispositif d'entraînement linéaire discontinu selon la revendication 3, **caractérisé en ce que** le dispositif de commande de déplacement (6b) ou la coulisse du dispositif d'entraînement (6) est réalisé(e) de manière qu'il ou elle déplace les composants de mise en prise d'un tout, et modifie la position mutuelle des éléments d'un composant de mise en prise.

5. Dispositif d'entraînement linéaire discontinu selon la revendication 3, **caractérisé en ce que** la coulisse est réalisée sous forme d'une pluralité de courbes formant came, réalisée sur la périphérie intérieure, en pourtour sur le dispositif d'entraînement (6) en forme de manchette, et avec un tronçon de glissement de forme correspondante, de préférence un organe d'entraînement de l'élément de mise en prise (3a) et de l'élément de serrage (3k), d'un composant de mise en prise identique, en contact avec glisssement.

6. Dispositif d'entraînement linéaire discontinu selon la revendication 3, **caractérisé en ce que** l'élément de mise en prise (3a) présente un tronçon annulaire (3b) et un tronçon de serrage (3d), le tronçon de serrage (3d) étant réalisé sous la forme d'une pluralité de doigts de serrage en forme de coin et flexibles, ressortant en direction axiale du tronçon annulaire (3b) et qui, du fait de leur forme en coin sont légèrement coudés radialement vers l'extérieur par rapport au tronçon annulaire (3b).

7. Dispositif d'entraînement linéaire discontinu selon la revendication 6, **caractérisé en ce que** l'élément de serrage (3k) est conformé en anneau, sur la périphérique intérieure duquel est prévue une pluralité de rainures (3n), dans lesquelles la pluralité des organes d'entraînement cunéiformes de élément de mise en prise (3a) est insérée.

8. Dispositif d'entraînement linéaire discontinu selon la revendication 7, **caractérisé en ce que** le composant tubulaire (2a) présente un dispositif de guidage (5), réalisé à la forme d'une pluralité d'évidements (5b), de préférence sous forme de fentes longitudinales, implanté(e)s de façon répartie sur un tronçon du composant tubulaire (2a), sur sa périphérie, dans lesquel(le)s la pluralité de doigts de serrage cunéiformes de l'élément de mise en prise (3a) est insérée.

9. Dispositif d'entraînement linéaire discontinu selon l'une des revendications précédentes, **caractérisé en ce que** celui-ci est entièrement réalisé en matière synthétique moulée par injection.

10. Dispositif d'entraînement linéaire discontinu selon l'une des revendications précédentes, **caractérisé en ce que** l'élément, qui est guidé au moyen du composant de guidage (2), réalisé sous forme de composant tubulaire (2a), est une construction à tube à retournement avec arbre d'endoscope, entraîné au moyen du dispositif d'entraînement linéaire.
